(19) Europäisches Patentamt
European Patent Office
Office européen des brevets

(11) **EP 3 248 586 B1**

(12) **EUROPEAN PATENT SPECIFICATION**

(45) Date of publication and mention
of the grant of the patent:
**29.05.2019 Bulletin 2019/22**

(51) Int Cl.:
***A61K 8/19*** *(2006.01)*          ***A61Q 19/10*** *(2006.01)*
***A61K 8/02*** *(2006.01)*

(21) Application number: **15807780.0**

(86) International application number:
**PCT/JP2015/071937**

(22) Date of filing: **03.08.2015**

(87) International publication number:
**WO 2016/117153 (28.07.2016 Gazette 2016/30)**

(54) **SCRUB AND METHOD FOR USING SAME**

SCHRUBBER UND VERFAHREN ZUR VERWENDUNG DAVON

EXFOLIANT ET SON PROCÉDÉ D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.01.2015 JP 2015010093**

(43) Date of publication of application:
**29.11.2017 Bulletin 2017/48**

(73) Proprietor: **MET CO., LTD.
Gamagori-shi,
Aichi 4430038 (JP)**

(72) Inventors:
• **TOYAMA, Tomitaka
Gamagori-shi, Aichi 443-0038 (JP)**
• **SAHASHI, Akihiro
Gamagori-shi, Aichi 443-0038 (JP)**
• **ITO, Takamitsu
Gamagori-shi, Aichi 443-0038 (JP)**
• **TOYAMA, Yuudai
Gamagori-shi, Aichi 443-0038 (JP)**

(74) Representative: **Kramer Barske Schmidtchen
Patentanwälte PartG mbB
European Patent Attorneys
Landsberger Strasse 300
80687 München (DE)**

(56) References cited:
JP-A- 2000 233 916      JP-A- 2002 212 020
JP-A- 2002 212 060      JP-A- 2006 083 052
JP-A- 2013 237 595      US-A1- 2005 061 147

**Description**

TECHNICAL FIELD

The present invention relates to a scrubbing agent and a method for using the same.

BACKGROUND ART

Skin care products such as face wash and body wash have been conventionally used for caring the skin of the face and body. Such skin care products usually contain a scrubbing agent for exfoliating old skin cells. As scrubbing agents, fine spherical plastic particles called microbeads have been frequently used for several years.
Incidentally, the precedent Patent Document 1 discloses a skin cleaner containing activated carbon produced using coconut shell as a raw material.

PRIOR ART DOCUMENT

Patent Document

Patent Document 1: JP-A-2001-233722

SUMMARY OF THE INVENTION

PROBLEM TO BE SOLVED BY THE INVENTION

However, the conventional technique suffers from the following drawback. Microbeads frequently used as scrubbing agents are fine as well as float on water. Thus, such microbeads, without captured in sewage sludge in sewage treatment facilities, are released through treated effluent discharges into the natural environment such as rivers, lakes, and oceans. Such microbeads released into the natural environment adsorb various chemical contaminants in the environment. The aquatic organisms mistake the microbeads having chemical contaminants adsorbed for food, and intake the microbeads into their body. Unfortunately, microbeads are prone to enter the food chain in this manner. Alternatively, when activated carbon made of coconut shell and the like is used as a scrubbing agent, its large surface asperities and asphericity may cause even necessary skin cell to be exfoliated.
The present invention has been made in the viewpoint of the above-mentioned background, and is intended to provide a scrubbing agent that facilitates exfoliation of old skin cells and is less prone to enter the food chain, and a method for using the scrubbing agent.

MEANS FOR SOLVING THE PROBLEM

One aspect of the present invention provides a scrubbing agent including an activated carbon:

wherein the activated carbon has a spherical shape, a median particle size in the range of 100 to 800 $\mu$m, a peak pore diameter in the range of 0.7 nm to 1.2 nm, and a micropore volume of not less than 0.5 cm$^3$/g, and
wherein the activated carbon satisfies a following Condition (1):
Condition (1): in a case when a sample of the activated carbon in an amount of one gram is distributed on a surface of 100 g of pure water having a temperature of 20°C and filled in a beaker with a depth of 5 cm; the beaker is placed in a vacuum desiccator; the vacuum desiccator is evacuated with a vacuum pump for 20 minutes; then, the pure water in the beaker is stirred with a stirring bar at a number of revolutions of 100 rpm for one minute; and the beaker is left to stand for 30 minutes, not less than 95% by mass of the sample precipitates on a bottom of the beaker.

Another aspect of the present invention provides a method for using the above mentioned scrubbing agent. The method includes a step of rolling the activated carbon on a skin surface.

EFFECTS OF THE INVENTION

The scrubbing agent contains activated carbon that is spherical and has a particle size in a specific range. Thus, old skin cell is relatively easily exfoliated by rolling the activated carbon contained in the scrubbing agent on the skin surface. In this case, the activated carbon is spherical, and thus can prevent the skin cells from being excessively exfoliated. In the scrubbing agent, the activated carbon has a peak pore diameter in the range of 0.7 nm to 1.2 nm and a micropore volume of not less than 0.5 cm$^3$/g. Thus, the scrubbing agent excels not only in the scrubbing function but also in the sebum adsorption capacity. Accordingly, while exfoliating old skin cells, the scrubbing agent can remove sebum by adsorption.

Also, the activated carbon in the scrubbing agent satisfies the specific condition (1). Thus, when the scrubbing agent is discharged along with water into the sewage, the activated carbon does not flow into the treated effluent (supernatant) but precipitates into the sewage sludge in the sewage treatment facility. Accordingly, the scrubbing agent can prevent its activated carbon having a scrubbing function from being released through treated effluent discharges into the natural environment such as rivers, lakes, and oceans. The scrubbing agent, therefore, is less prone to enter the food chain.

The method for using the scrubbing agent includes a procedure for rolling the activated carbon contained in the scrubbing agent on the skin surface. The method for using the scrubbing agent, thus, allows the activated carbon to exfoliate old skin cells relatively easily. Additionally, while exfoliating old skin cells, the method can also remove sebum by adsorption. Even when the scrubbing agent is discharged along with water into the sewage after use, the activated carbon precipitates into and is captured by the sewage sludge in the sewage treatment facility, and thus, is less prone to be released into the natural environment as described above. The method for using the scrubbing agent thus enables natural environment-friendly skin care to be achieved because the scrubbing agent is less prone to enter the food chain.

Therefore, the present invention can provide a scrubbing agent that facilitates exfoliation of old skin cells and is less prone to enter the food chain, and a method for using the scrubbing agent.

BREIF DESCRIPTION OF THE DRAWINGS

Figure 1 is a schematic illustration showing a scrubbing agent according to Example 1.
Figure 2 is a schematic illustration showing a scrubbing agent according to Example 2.
Figure 3 is the differential pore volume distribution of each activated carbon and Carbide C in Experimental Example.
Figure 4 is a photograph showing the state of each sample bottle after shaking observed in the oleic acid adsorption test in Experimental Example.

MODE FOR CARRYING OUT THE INVENTION

In the scrubbing agent, the activated carbon has a spherical shape. Spherical means not only a true sphere, but also a shape equivalent to a sphere, that is, a shape similar to a sphere. Specifying that the activated carbon is spherical is significant in that the activated carbon smoothly rolls on the skin surface to exfoliate old skin cells by the scrubbing action and to cause less damage to the necessary skin cells.

The activated carbon has a median particle size in the range of 100 to 800 $\mu$m. When activated carbon has a median particle size less than 100 $\mu$m, such activated carbon increasingly penetrates into human follicles and may become less prone to smoothly roll on the skin surface. As the result, the feeling of use of the scrubbing agent will be deteriorated. Additionally, when the scrubbing agent includes activated carbon singly, such activated carbon is prone to be scattered as described below. From the viewpoint of decreasing such a disadvantage, the median particle size of the activated carbon may be preferably not less than 110 $\mu$m, more preferably not less than 120 $\mu$m, still more preferably not less than 150 $\mu$m, even more preferably not less than 180 $\mu$m, and still even more preferably not less than 200 $\mu$m. In contrast, when activated carbon has a median particle size more than 800 $\mu$m, a user of the scrubbing agent may be prone to feel discomfort and pain in use. From the viewpoint of decreasing such a disadvantage, the median particle size of the activated carbon may be preferably not more than 750 $\mu$m, more preferably not more than 700 $\mu$m, still more preferably not more than 650 $\mu$m, even more preferably not more than 600 $\mu$m, still even more preferably not more than 550 $\mu$m, and most preferably not more than 500$\mu$m.

The median particle size of the activated carbon refers to the particle size (diameter) d50 when the cumulative frequency distribution on a volume basis measured by a laser diffraction/scattering particle size distribution analyzer ("LA-700" manufactured by HORIBA, Ltd.) is 50%. When the particle size distribution analyzer becomes unavailable, a particle size distribution analyzer that enables the comparable measurement to be carried out is used.

The activated carbon hereby satisfies the following condition (1).
Condition (1): in a case when a sample of the above mentioned activated carbon in an amount of one gram is distributed on the surface of 100 g of pure water having a temperature of 20°C and filled in a beaker with a depth of 5 cm; the beaker is placed in a vacuum desiccator; the vacuum desiccator is evacuated with a vacuum pump for 20 minutes; then, the pure water in the beaker is stirred with a stirring bar at a number of revolutions of 100 rpm for one minute; and the beaker is left to stand for 30 minutes, not less than 95% by mass of the sample precipitates on a bottom of the beaker.

Satisfying the condition (1) by the activated carbon in the scrubbing agent is significant in that most activated carbon contained in the scrubbing agent is submerged in water and is recovered into the sewage sludge in the sewage treatment facility. In addition to activated carbon having a specific gravity of less than 1, activated carbon having a specific gravity more than 1 may also float on water if the activated carbon particles contain many closed pores therein. Thus, it is impossible to accurately determine whether or not most activated carbon in contained in a scrubbing agent is submerged in water by the magnitude of the specific gravity. Meanwhile, with respect to the activated carbon that satisfies the condition (1), most activated carbon particles are submerged in water. Even if pores open to the activated carbon surface are filled with air, the air escapes from the pore during flowing in the sewerage or by agitation and the like in the sewage treatment facility. Then, the activated carbon will be submerged in water and recovered into the sewage sludge.

Under the condition (1), an activated carbon sample is distributed on the surface of the water in the beaker. The beaker is placed in a vacuum desiccator, and then the vacuum desiccator is evacuated with a vacuum pump. Accordingly, the air that has penetrated into the pores of the activated carbon of the sample is completely removed. In other words, it is possible to accurately determine whether or not most activated carbon contained in a scrubbing agent is submerged in the water in a condition that the influence of the air that has penetrated into the pores is eliminated. Incidentally, the ultimate pressure is from 10 to 50 Pa. The activated carbon that floats on water without precipitating on the bottom of the beaker is allowed to be contained up to 5% by mass on the basis of the sample.

In the scrubbing agent, the peak pore diameter of the activated carbon is a pore diameter at which the differential pore volume distribution of each activated carbon exhibits the maximum value.

From the viewpoint of improving the sebum adsorption capacity and the like, the peak pore diameter of the activated carbon may be preferably not less than 0.75 nm, more preferably not less than 0.8 nm, still more preferably not less than 0.85 nm, and even more preferably not less than 0.9 nm. From the viewpoint of achieving the mechanical strength of the activated carbon and the like, the peak pore diameter of the activated carbon may be preferably not more than 1.18 nm, more preferably not more than 1.15 nm, and still more preferably not more than 1.13 nm.

The differential pore volume distribution and micropore volume of the activated carbon can be obtained as follows. In the pretreatment, the activated carbon is maintained under a condition of 300°C and less than $10^{-2}$ kPa for two hours by use of a pretreatment device ("BELSORP-VACII" manufactured by BEL JAPAN, INC.). Subsequently, an automatic specific surface area/pore size distribution analyzer ("BELSORP-mini II" manufactured by BEL JAPAN, INC.) is used to form an adsorption isotherm showing the variation in the amount of nitrogen gas adsorbed against the pressure at a constant temperature (77K) to thereby provide the differential pore volume distribution and micropore volume by calculation in the MP method.

In the scrubbing agent, the activated carbon may have a specific surface area of not less than 1000 $m^2$/g. In this case, there is provided a scrubbing agent that excels not only in the scrubbing function but also in the sebum adsorption capacity. Accordingly, while exfoliating old skin cells, the scrubbing agent removes sebum by adsorption.

From the viewpoint of improving the sebum adsorption capacity and the like, the specific surface area of the activated carbon may be preferably not less than 1100 $m^2$/g, more preferably not less than 1200 $m^2$/g, still more preferably not less than 1300 $m^2$/g, and even more preferably not less than 1400 $m^2$/g. Incidentally, from the viewpoint of increasing sebum adsorption sites, the larger the specific surface area of the activated carbon, the better. However, the specific surface area of the activated carbon is excessively increased, the yield of the activated carbon is reduced to impair the economic efficiency. Additionally, the mechanical strength of the activated carbon tends to decrease. Thus, the specific surface area of the activated carbon is preferably not more than 2400 $m^2$/g.

The specific surface area of the activated carbon can be obtained as follows. The activated carbon is pretreated by maintaining at 300°C and less than $10^{-2}$ kPa for two hours by use of a pretreatment device ("BELSORP-VACII" manufactured by BEL JAPAN, INC.). Subsequently, an automatic specific surface area/pore size distribution analyzer ("BELSORP-mini II" manufactured by BEL JAPAN, INC.) is used to form an adsorption isotherm showing the variation in the amount of nitrogen gas adsorbed against the pressure at a constant temperature (77K) to thereby provide the specific surface area by calculation in the BET method in compliant with ISO 9277. Incidentally, in the description above, when the automatic specific surface area/pore size distribution analyzer becomes unavailable, an automatic specific surface area/pore size distribution analyzer that enables the comparable measurement to be carried out is used.

The scrubbing agent may include no surfactant. Surfactants are prone to be adsorbed into pores of the activated carbon. Thus, when an excess amount of a surfactant is contained, the pores of the activated carbon are occupied by the surfactant, and the sebum adsorption capacity of the activated carbon may be reduced accordingly. In the case where the scrubbing agent includes no surfactant, if the above mentioned scrubbing agent is used singly, it becomes easy to maximize the sebum adsorption capacity of the activated carbon because the capacity is not inhibited. Incidentally, the surfactant mentioned above refers to those which serve to emulsify and dissolve human sebum in water. Examples of such surfactants include sodium lauryl sulfate and PEG-20 glyceryl oleate.

For example, the scrubbing agent may be configured to include the activated carbon as a single component. In this case, it is easy to maximize the scrubbing function to exfoliate old skin cells and the sebum adsorption function, which are functions of the activated carbon.

The scrubbing agent may be configured to further include a base into which the activated carbon is dispersed, in addition to the activated carbon. In this case, when the scrubbing agent is placed on a hand, the base allows the activated carbon to be easily maintained in the hand. Thus, there is provided a scrubbing agent advantageous for improving the handleability and the feeling of use in this case. The base may be a liquid, or a semi-solid such as a paste. Examples of the base may include water, carboxymethyl cellulose gel, and those contained in ordinary skin care products. A base that contains no surfactant can be suitably used. In this case, it is easy to maximize the scrubbing function to exfoliate old skin cells and the sebum adsorption function, which are provided by the activated carbon.

When the base is water, there is provided an aqueous scrubbing agent, which has little irritation caused by the base to the skin and has a lighter load on the natural environment.

The scrubbing agent can be produced as follows, for example. Spherical resin raw material powder is carbonized in a carbonizing furnace to provide spherical carbide powder. As the spherical resin raw material powder, spherical phenol resin powder can be used, for example. Incidentally, the median particle size d50 of the spherical resin raw material powder may be from of the order of 140 to 1100 $\mu$m. In an exemplary carbonization condition, the powder can be maintained under nitrogen atmosphere at a temperature of 850°C for 30 minutes. Then, the carbonized powder obtained is activated in an activating furnace. In an exemplary activation condition, water vapor can be allowed to flow into the furnace, in which the powder can be maintained at a temperature of 850°C for 5 to 24 hours. Additionally, the activated carbon obtained by activation may be classified so as to have a predetermined particle size, as required. This can provide activated carbon powder that can be used for the scrubbing agent.

The method for using the scrubbing agent includes a step of rolling the activated carbon on the skin surface. In the method for using the scrubbing agent, specifically, for example, the scrubbing agent placed on a palm is brought into contact with a predetermined skin surface to sandwich the activated carbon between the palm and the predetermined skin surface. The hand maintained in this state is moved to enable the activated carbon to roll on the skin surface.

Incidentally, each component described above may be optionally combined as required, for example, in order to achieve each function and effect described above.

EXAMPLES

The scrubbing agents of Examples will be described hereinbelow, referring to the drawings. The same number will be described using the same reference number.

(Example 1)

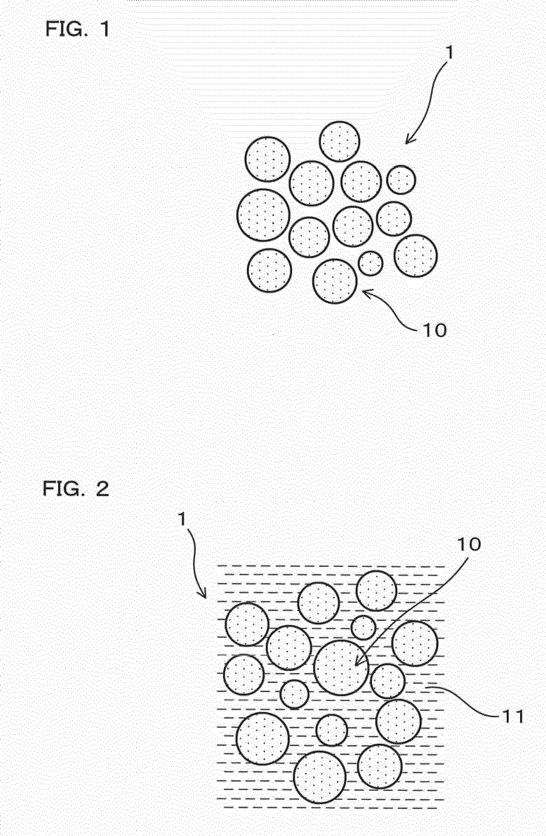

The scrubbing agent of Example 1 will be described, referring to Figure 1. As shown in Figure 1, the scrubbing agent 1 of the present example includes activated carbon 10. In the present example, the scrubbing agent 1 includes activated carbon 10 singly.

The activated carbon 10 in this example has a spherical shape, a median particle size in the range of 100 to 800 $\mu$m. Additionally, the activated carbon 10 has a peak pore diameter in the range of 0.7 nm to 1.2 nm and a micropore volume of not less than 0.5 cm$^3$/g. Also, the activated carbon 10 satisfies the following condition (1). Condition (1): in a case when a sample of the above mentioned activated carbon 10 in an amount of one gram is distributed on the surface of 100 g of pure water having a temperature of 20°C and filled in a beaker with a depth of 5 cm; the beaker is placed in a vacuum desiccator; the vacuum desiccator is evacuated with a vacuum pump for 20 minutes; then, the pure water in the beaker is stirred with a stirring bar at a number of revolutions of 100 rpm for_ one minute; and the beaker is left to stand for 30 minutes, not less than 95% by mass of the sample precipitates on the bottom of the beaker.

In the present example, the activated carbon 10 has a specific surface area of not less than 1000 m$^2$/g.

Subsequently, the functions and effects of the scrubbing agent of the present example will be described.

The scrubbing agent 1 of the present example has the activated carbon 10, which is spherical and has a particle size in a specific range. Thus, old skin cell is relatively easy to exfoliate by rolling the activated carbon 10 contained in the scrubbing agent 1 of the present example on the skin surface. In this case, the activated carbon 10 is spherical, and thus can prevent excess exfoliation of skin cells.

Also, the activated carbon 10 in the scrubbing agent 1 of the present example additionally satisfies the specific condition (1). Thus, when the scrubbing agent 1 of the present example is discharged along with water into the sewage, the activated carbon 10 does not flow into the treated effluent (supernatant) but precipitates into the sewage sludge in the sewage treatment facility. Accordingly, the scrubbing agent 1 of the present example can prevent the activated carbon 10 having a scrubbing function from being released via treated effluent discharges into the natural environment such as rivers, lakes, and oceans. The scrubbing agent 1 of the present example, therefore, is less prone to enter the food chain.

Additionally, the activated carbon 10 in the scrubbing agent 1 of the present example has a peak pore diameter, a micropore volume, and a specific surface area in the range described above. Thus, the scrubbing agent 1 of the present example excels not only in the scrubbing function but also in the sebum adsorption capacity.

Additionally, the scrubbing agent 1 of the present example includes activated carbon 10 singly. Thus, it is easy for the scrubbing agent 1 of the present example to maximize the scrubbing function to exfoliate old skin cells and the sebum adsorption function, which are provided by the activated carbon 10. Furthermore, in the present example, powder scrubbing agent 1 can be obtained.

(Example 2)

The scrubbing agent of Example 2 will be described, referring to Figure 2. The scrubbing agent 1 of the present example is different from the scrubbing agent 1 of Example 1 in that the scrubbing agent 1 of the present example includes activated carbon 10 and a base 11 into which the activated carbon 10 is to be dispersed. In the present example, the base 11 is specifically water. Other components are the same as those of Example 1.

The scrubbing agent 1 of the present example, as the scrubbing agent 1 of Example 1, facilitates exfoliation of old skin cells and is less prone to enter the food chain. The scrubbing agent 1 excels not only in the scrubbing function but also in the sebum adsorption capacity.

Additionally, the scrubbing agent 1 of the present example includes, in addition to the activated carbon 10, the base 11 into which the activated carbon 10 is to be dispersed. Thus, when the scrubbing agent 1 of the present example is placed on a hand, the base 11 allows the activated carbon 10 to be easily maintained in the hand. Thus, the scrubbing agent 1 of the present example is advantageous for improving the handleability and the feeling of use. Additionally, since the base 11 is water in the present example, there is provided an aqueous scrubbing agent 1, which has little irritation caused by the base 11 to the skin and has a lighter load on the natural environment.

(Experimental Example)

More specific description will be given hereinbelow, referring to Experimental Example.

<Production of activated carbon AC1, AC2, and AC3>

Spherical phenol resin powder having a median particle size of 250 $\mu$m was subjected to carbonization, in which the powder was heated under nitrogen atmosphere at a temperature rising rate of 3°C/minute to 850°C and maintained for 30 minutes in the carbonizing furnace of activated carbon manufacturing equipment (manufactured by MET Inc.), to thereby provide spherical carbonized powder. Then, the obtained spherical carbonized powder was subjected to activation, in which the powder was heated at a temperature rising rate of 3°C/minute to 850°C in the activating furnace of the activated carbon manufacturing equipment followed by allowing water vapor at 12 g/minute to flow into the furnace, and the powder was maintained for 5 hours, 10 hours, and 24 hours. This provided spherical activated carbon powders AC3 at a yield of 31.1%, AC2 at a yield of 25%, and AC1 at a yield of 7%, each corresponding to the above mentioned maintenance times. Incidentally, the yields refer to the ratio of the mass of the obtained activated carbon (%) to the mass of the raw material in the absolute dried state.

<Production of Carbide C>

The spherical carbonized powder obtained in the course of the production of the above mentioned activated carbon was used as Carbide C.

<Production of Japanese cedar carbide BC>

Japanese cedar chips were dried at 120°C to a water content of not more than 12% by mass. Then, the dried Japanese cedar chips were pelletized by using biomass pellet manufacturing equipment (manufactured by Earth Engineering Corporation, "EF-BS-150") to thereby provide Japanese cedar pellets. Then, the obtained Japanese cedar pellets were subjected to carbonization, in which the pellets were heated under nitrogen atmosphere at a temperature rising rate of 3°C/minute to 850°C and maintained for 30 minutes in the carbonizing furnace of the activated carbon manufacturing equipment. Then, the obtained Japanese cedar carbide was pulverized in a mortar to thereby provide non-spherical particulate Japanese cedar carbide BC.

<Particle size measurement>

The median particle size d50 of each of activated carbon, Carbide C and Japanese cedar carbide BC was measured using a laser diffraction/scattering particle size distribution analyzer ("LA-700" manufactured by HORIBA, Ltd.) The particle size (diameter) d90 when the cumulative frequency distribution on a volume basis is 90% was also measured.

<Test for determining whether or not the condition (1) is satisfied>

A beaker containing 100 g of pure water at a temperature of 20°C up to a height of 5 cm was provided. Then, one gram of a sample of activated carbon to be measured was distributed on the surface of the water surface of the beaker, and the beaker was placed in a 9 L vacuum desiccator. Then, the desiccator was evacuated using a vacuum pump (manufactured by ULVAC KIKO, Inc., "DA-5S") for 20 minutes to thereby remove air in the pores of the activated carbon floating on the water surface. Incidentally, the ultimate pressure was 33.3 Pa. Then, after vacuum was released, the beaker was taken out of the vacuum desiccator. Then, the pure water in the beaker was stirred with a stirring bar at a number of revolutions of 100 rpm for one minute, and the beaker was left to stand for 30 minutes. The case where not less than 95% by mass of the sample precipitated on the bottom of the beaker was determined to satisfy the condition (1). The other cases were determined not to satisfy the condition (1). Incidentally, Carbide C and Japanese cedar carbide BC, which were not activated carbon, were omitted to this determination test.

<Differential pore volume distribution of each activated carbon and Carbide C>

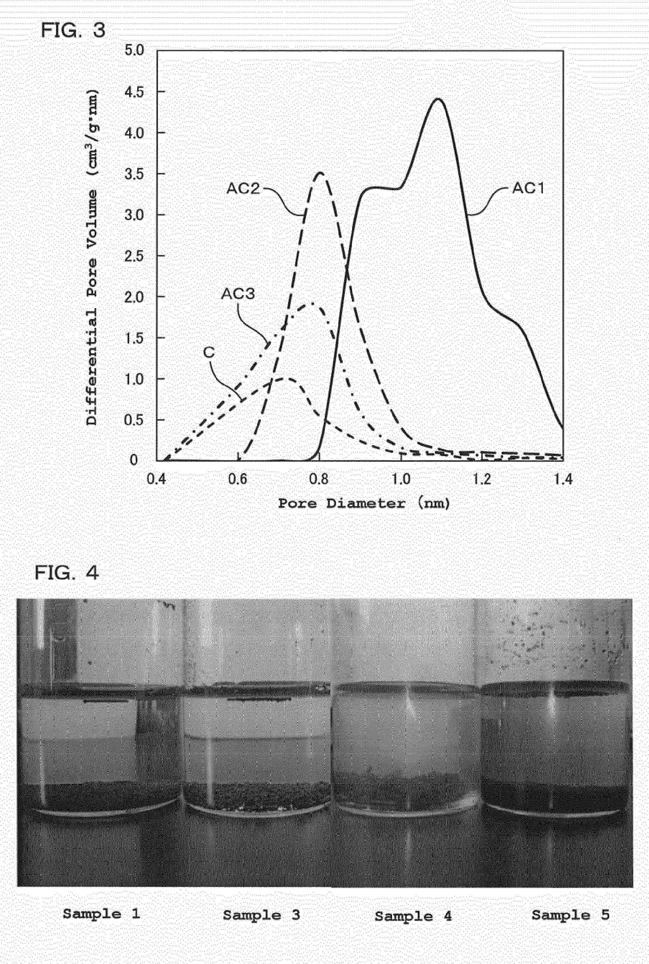

Each of activated carbon and Carbide C was subjected to pretreatment, in which the activated carbon or Carbide C was maintained at 300°C and less than $10^{-2}$ kPa for two hours by use of a pretreatment device ("BELSORP-VACII" manufactured by BEL JAPAN, INC.). Then, an automatic specific surface area/pore size distribution analyzer ("BELSORP-mini II" manufactured by BEL JAPAN, INC.) was used to form an adsorption isotherm showing the variation in the amount of nitrogen gas adsorbed against the pressure at a constant temperature (77K). Subsequently, the differential pore volume distribution and micropore volume were determined by the MP method. The differential pore volume distribution of each activated carbon and Carbide C is shown in Figure 3. Incidentally, it was not possible to analyze Japanese ceder carbide BC because the carbide had almost no micropore pores.

<Specific surface area of each activated carbon, Carbide C, and Japanese cedar carbide BC>

Each of activated carbon, Carbide C, and Japanese cedar carbide BC was pretreated by maintaining at 300°C and less than $10^{-2}$ kPa for two hours by use of a pretreatment device ("BELSORP-VACII" manufactured by BEL JAPAN, INC.). Then, an automatic specific surface area/pore size distribution analyzer ("BELSORP-mini II" manufactured by BEL JAPAN, INC.) was used to form an adsorption isotherm showing the variation in the amount of nitrogen gas adsorbed against the pressure at a constant temperature (77K). Subsequently, the specific surface area was determined by the BET method in compliant with ISO 9277.

<Scrubbing agents of Samples 1 to 5 >

The activated carbon AC1 was used as the scrubbing agent of Sample 1. The activated carbon AC2 was used as the scrubbing agent of Sample 2. The activated carbon AC3 was used as the scrubbing agent of Sample 3. Carbide C was used as the scrubbing agent of Sample 4. Japanese cedar carbide BC was used as the scrubbing agent of Sample 5. Incidentally, the scrubbing agents of Samples 4 and 5 were Reference Examples.

<Oleic acid adsorption test>

The scrubbing agents of Samples 1 to 5 were measured for the oleic acid adsorption ratio. Incidentally, oleic acid was used to simulate sebum. In other words, this test enables to confirm the sebum adsorption capacity of the scrubbing agents of the Samples 1 to 5.

An aqueous solution containing 1000 ppm oleic acid on the basis of the mass ratio was prepared and sufficiently stirred. Then, 50 g of the above mentioned aqueous solution and one gram (absolute dried weight) of the scrubbing agent of Sample were placed in this order in a sample bottle, which was then sealed. Then, the sealed sample bottle was shaken using a shaker ("RECIPRO SHAKER NR-1" manufactured by TAITEC CORPORATION) under a condition of a temperature: 35°C, a shaking rate: 150 reciprocations/minute, and a shaking time: 24 hours. Then, the transparency of the sample bottle after shaking was visually observed. The state of each sample bottle after shaking is shown in Figure 4. Then, the liquid in the sample bottle was filtered to remove the sample scrubbing agent, which was allowed to stand at a temperature of 25°C for one day to be dried. Then, the sample scrubbing agent after drying was analyzed using a thermogravimetric analyzer ("THERMO PLUS TG8120" manufactured by Rigaku Corporation). The analysis condition for this case included maintaining the sample at 105°C for two hours under nitrogen atmosphere, at a temperature rising rate of 2°C/minute followed by heating the sample to 650°C. Then, the adsorption ratio of oleic acid (%) was calculated by the following calculation expression.

$$\text{Adsorption ratio of oleic acid (\%)} = \frac{(\text{Weight loss at 600°C} - \text{Weight loss at 105°C}) \times 100}{(\text{Absolute dried weight of the sample scrubbing agent})}$$

The above mentioned results are summarized in Table 1.

[Table 1]

[Table 1]

| Samples | | Shape | Particle size ($\mu$m) | | Micropore pore volume (cm3/g) | Peak pore diameter (nm) | Specific surface area (m2/g) | Condition (1) | Oleic acid adsorption ratio (%) |
|---|---|---|---|---|---|---|---|---|---|
| | | | Median particle size d50 | Cumulative frequency distribution 90% d90 | | | | | |
| Sample 1 | Activated carbon AC1 (Yield: 7%) | Spherical | 125.4 | 171.6 | 1.217 | 1.09 | 2230 | Satisfied | 6.36 |
| Sample 2 | Activated carbon AC2 (Yield: 25%) | Spherical | 149.2 | 195.5 | 0.707 | 0.80 | 1590 | Satisfied | 6.09 |
| Sample 3 | Activated carbon AC3 (Yield: 31.1%) | Spherical | 155.3 | 201.1 | 0.539 | 0.79 | 1262 | Satisfied | 4.77 |
| Sample 4 | Carbide C | Spherical | 190.7 | 210.5 | 0.204 | 0.72 | 476 | - | 1.03 |
| Sample 5 | Japanese ceder carbide BC | Non-spherical | 570.4 | 610.5 | Unmeasurable | Unmeasurable | $\leqq 100$ | - | 3.27 |

The scrubbing agents of Samples 1 to 3 include activated carbon which is spherical and has a particle size in a specific range. Additionally, the scrubbing agents of Samples 1 to 3 have an oleic acid adsorption ratio higher than that of the scrubbing agents of Samples 4 and 5. Thus, the scrubbing agents of Samples 1 to 3, while exfoliating old skin cells by the scrubbing function of the activated carbon, can remove sebum by adsorption. Incidentally, as shown in Figure 4, the sample bottle containing the scrubbing agent of Sample 1 was transparent, and the sample bottle containing the scrubbing agent of Sample 3 was substantially transparent although slight cloudiness was observed. In contrast, the sample bottles each containing the scrubbing agents of Sample 4 and Sample 5 were so clouded that the interior of the sample bottles could not be sufficiently observed. This is because the samples could not sufficiently adsorb the oleic acid. The above mentioned results are consistent with the results of the oleic acid adsorption ratio measurement.

Also, the activated carbon in the scrubbing agents of Samples 1 to 3 satisfies the condition (1). Thus, when each scrubbing agent is discharged along with water into the sewage, the activated carbon does not flow into the treated effluent (supernatant) but precipitates into the sewage sludge in the sewage treatment facility. Accordingly, the scrubbing agent can prevent its activated carbon having a scrubbing function from being released via release of treated effluent into the natural environment such as rivers, lakes, and oceans. It can be said that the scrubbing agent, therefore, is less prone to enter the food chain. Incidentally, microbeads, which have been conventionally used as scrubbing agents, clearly floats on water, and thus, clearly fail to satisfy the condition (1). Moreover, microbeads themselves clearly have no sebum adsorption capacity.

## Claims

1. A scrubbing agent comprising an activated carbon:

   wherein the activated carbon has a spherical shape, a median particle size in the range of 100 to 800 $\mu$m, a peak pore diameter in the range of 0.7 nm to 1.2 nm, and a micropore volume of not less than 0.5 cm$^3$/g, and wherein the activated carbon satisfies a following Condition (1):
   Condition (1): in a case when a sample of the activated carbon in an amount of one gram is distributed on a surface of 100 g of pure water having a temperature of 20°C and filled in a beaker with a depth of 5 cm; the beaker is placed in a vacuum desiccator; the vacuum desiccator is evacuated with a vacuum pump for 20 minutes; then, the pure water in the beaker is stirred with a stirring bar at a number of revolutions of 100 rpm for one minute; and the beaker is left to stand for 30 minutes, not less than 95 % by mass of the sample precipitates on a bottom of the beaker.

2. The scrubbing agent according to claim 1, wherein the activated carbon has a specific surface area of not less than 1000 m$^2$/g.

3. The scrubbing agent according to claim 1 or 2, comprising the activated carbon as a single component.

4. The scrubbing agent according to claim 1 or 2, further comprising a base into which the activated carbon is dispersed.

5. The scrubbing agent according to any one of claims 1 to 4, comprising no surfactant.

6. A method for using the scrubbing agent according to any one of claims 1 to 5, comprising a step of rolling the activated carbon on a skin surface.

## Patentansprüche

1. Peelingmittel umfassend eine Aktivkohle:

   wobei die Aktivkohle eine Kugelform, eine mittlere Teilchengröße in dem Bereich von 100 bis 800 $\mu$m, einen Spitzenporendurchmesser in dem Bereich von 0,7 nm bis 1,2 nm und ein Mikroporenvolumen von nicht weniger als 0,5 cm$^3$/g aufweist, und
   wobei die Aktivkohle eine folgende Bedingung (1) erfüllt:
   Bedingung (1): in einem Fall, wenn eine Probe der Aktivkohle in einer Menge von einem Gramm über eine Oberfläche von 100 g an reinem Wasser mit einer Temperatur von 20 °C verteilt und in ein Becherglas mit einer Tiefe von 5 cm gefüllt wird; das Becherglas in einen Vakuumexsikkator gestellt wird; der Vakuumexsikkator mit einer Vakuumpumpe 20 Minuten lang evakuiert wird; dann das reine Wasser in dem Becherglas mit einem Rührer bei einer Drehzahl von 100 U/min eine Minute lang gerührt wird; und das Becherglas 30 Minuten lang stehengelassen wird, wobei nicht weniger als 95 Gew.-% der Probe auf einem Boden des Becherglases ausfällt.

2. Peelingmittel nach Anspruch 1, wobei die Aktivkohle eine spezifische Oberfläche von nicht weniger als 1000 m$^2$/g aufweist.

3. Peelingmittel nach Anspruch 1 oder 2,
umfassend die Aktivkohle als eine einzelne Komponente.

4. Peelingmittel nach Anspruch 1 oder 2, ferner umfassend einen Grundstoff, in dem die Aktivkohle dispergiert ist.

5. Peelingmittel nach einem der Ansprüche 1 bis 4, welches kein Tensid umfasst.

6. Verfahren zur Verwendung des Peelingmittels nach einem der Ansprüche 1 bis 5, umfassend einen Schritt des Rollens der Aktivkohle auf eine Hautoberfläche.

**Revendications**

1. Agent exfoliant comprenant un charbon activé :

dans lequel le charbon activé a une forme sphérique, une taille de particule médiane dans la plage de 100 à 800 $\mu$m, un diamètre de pic de pores dans la plage de 0,7 nm à 1,2 nm, et un volume de micropore non inférieur à 0,5 cm$^3$/g, et
dans lequel le charbon activé satisfait à une Condition (1) suivante :
Condition (1) : dans un cas où un échantillon de charbon activé en une quantité d'un gramme est réparti sur une surface de 100 g d'eau pure ayant une température de 20°C et rempli dans un bécher d'une profondeur de 5cm; le bécher est placé dans un dessiccateur sous vide ; le dessiccateur sous vide est mis sous vide à l'aide d'une pompe à vide pendant 20 minutes ; ensuite, l'eau pure dans le bécher est agitée à l'aide d'un agitateur à un nombre de révolutions à 100 tr/min pendant une minute; et le bécher est laissé au repos pendant 30 minutes, pas moins de 95% en masse de l'échantillon précipitent dans un fond du bécher.

2. Agent exfoliant selon la revendication 1, dans lequel le charbon activé a une surface spécifique non inférieure à 1000 m$^2$/g.

3. Agent exfoliant selon la revendication 1 ou 2,
comprenant le charbon activé en tant que composant unique.

4. Agent exfoliant selon la revendication 1 ou 2, comprenant en outre une base dans laquelle le charbon activé est dispersé.

5. Agent exfoliant selon l'une quelconque des revendications de 1 à 4, ne comprenant pas d'agent tensioactif.

6. Procédé d'utilisation de l'agent exfoliant selon l'une quelconque des revendications 1 à 5, comprenant une étape consistant à rouler le charbon actif sur une surface de peau.

FIG. 1

FIG. 2

FIG. 3

FIG. 4

Sample 1        Sample 3        Sample 4        Sample 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001233722 A